# EUROPEAN PATENT SPECIFICATION

(11) **EP 2 214 643 B1**
(45) Date of publication and mention of the grant of the patent: **02.04.2014**
(21) Application number: 08845547.2
(22) Date of filing: 31.10.2008
(51) Int. Cl.: A61K 9/00, A61K 47/32, A61K 31/56, A61K 31/57, A61K 47/14, A61K 9/12, A61K 47/10

(54) **TRANSDERMAL DELIVERY SYSTEM FOR HORMONES AND STEROIDS**
TRANSDERMALES FREISETZUNGSSYSTEM FÜR HORMONE UND STEROIDE
SYSTÈME D'ADMINISTRATION TRANSDERMIQUE POUR HORMONES ET STÉROÏDES

(30) Priority: 02.11.2007 US 984787 P
(43) Date of publication of application: 11.08.2010
(73) Proprietor: Acrux DDS Pty Ltd, West Melbourne, Victoria 3003 (AU)
(72) Inventor: SETIAWAN, Kerrie, West Melbourne, Victoria 3003 (AU); WATKINSON, Adam, West Melbourne, Victoria 3003 (AU)
(74) Representative: Schnappauf, Georg
(86) International application number: PCT/AU2008/001613
(87) International publication number: WO 2009/055859

(56) References cited:
- EP-A2- 0 328 806
- EP-A2- 0 409 383
- WO-A1-00/44347
- WO-A1-93/10201
- WO-A1-94/06452
- WO-A1-94/07478
- WO-A1-03/039597
- WO-A1-2007/016766
- WO-A2-00/45795
- US-A1- 2006 275 218
- US-B2- 6 818 226
- MORGAN, T.M. ET AL.: 'Transdermal delivery of estradiol in postmenopausal women with a novel topical aerosol' J. PHARMA. SCI. vol. 87, no. 10, 1998, pages 1226 - 1228, XP000777994
- MORGAN, T.M. ET AL.: 'Enhanced Transdermal delivery of sex hormones in swine with a novel topical aerosol.' J. PHARMA. SCI. vol. 87, no. 10, 1998, pages 1219 - 1225, XP000777993
- OSBOURNE, DW ET AL.: 'Skin penetration Enhancers cited in the technical literature' PHARMACEUTICAL TECHNOLOGY. November 1997, pages 58 - 66, XP008134247
- H.-Y. THONG ET AL.: 'Percutaneous Penetration Enhancers: An Overview.' SKIN PHARMACOL PHYSIOL. vol. 20, 2007, pages 272 - 282, XP009139652

## Description

### Field

This invention relates to a transdermal delivery system and to a transdermal delivery of hormones and steroids.

### Background

Steroids and hormones include sex hormones and certain adrenocortical hormones (corticosteroids). The corticosteroids have numerous and diversified physiological functions and pharmacological effects. They influence carbohydrate, protein, fat, and purine metabolism; electrolyte and water balance; and the functions of the cardiovascular system, the kidney, skeletal muscle, nervous system, and other organs and tissues. Therapeutically, the corticosteroids are used for treating hormonal insufficiencies, inflammation, and other conditions, whereas the sex hormones are widely used for contraception and hormonal insufficiencies, as well as for treating other conditions.

The two main classes of sex steroids are androgens and estrogens, of which the most important human derivatives are testosterone and estradiol (17p-estradiol), respectively. Other contexts will Include progestagen as a third class of sex steroids, distinct from androgens and estrogens. Progesterone is the only naturally-occurring human progestagen. Progestins are synthetic sex hormones used in contraception either alone or with estradiol. Androgens are often referred to as "male sex hormones", since they have masculinizing effects, while estrogens and progestagens are considered "female sex hormones" although all types are present in each gender, albeit at different levels. Androgens may be used in treatment of reduced libido or in treatment of depression in both men and women.

Administration of hormones and steroids through the skin ('transdermal delivery') has received increased attention because it not only provides a potentially simple dosage regime but it also provides a relatively controlled route for release of a hormone into the systemic circulation. However, transdermal drug delivery is complicated by the fact that the skin behaves as a natural barrier and therefore transport of agents through the skin is a complex mechanism.

Structurally, the skin consists of two principle parts, a relatively thin outermost layer (the 'epidermis') and a thicker inner region (the 'dermis'). The outermost layer of the epidermis (the 'stratum corneum') consists of flattened dead cells which are filled with keratin. The region between the flattened dead cells of the stratum corneum is filled with lipids which form lamellar phases that are responsible for the natural barrier properties of the skin.

For effective transdermal delivery of a pharmacological agent that is applied to the surface of the skin ('topical application'), the agent must partition firstly from the vehicle into the stratum corneum, it must typically then diffuse within the stratum corneum before partitioning from the stratum corneum to the viable epidermis.

A transdermal "patch" typically consists of a matrix or reservoir containing the drug to be administered, together with a backing layer, an adhesive and a protective release liner. Release membranes may also be incorporated. The delivery of drugs through these systems is either through passive diffusion, controlled by a semi-permeable release membrane, or is controlled by the adhesive/adhesive matrix. The system may also incorporate drug penetration enhancers to increase the flux of the drug through the skin.

One of the drawbacks of the current approaches to administering hormones and steroids is that the formulations are typically in continuous contact with the skin. Creams and ointments or adhesives used in patches can cause skin irritation and sensitisation. A significant proportion of patch users suffer from skin irritation and sensitisation due to adhesives used in the patch. Steroids and hormones, particularly the sex steroidal hormones have a relatively poor skin permeation and many patches require high loads of drug or large a surface area in order to provide effective blood levels.

The rate of drug delivery across a dermal surface can be increased by dermal penetration enhancers. The problem with most known dermal penetration enhancers is that they are often toxic, irritating or allergenic. These enhancers tend to be proton accepting solvents such as dimethylsulfoxide and dimethylacetamide. More recently, 2-pyrrolidine, *N*,*N*-diethyl-*m-*toluamide (Deet), 1-dodecal-azacycloheptane-2-one (Azone), *N, N* dimethylformamide, N-methyl-2-pyrrolidine and calcium thioglycolate have been reported as effective enhancers. However, difficulties remain with because the problem of irritation at the site of application. and/or difficulty in providing sufficient enhancement of transdermel absorption.

WO 94/06452 discloses a suspension of protein or peptide in triacetin or polyethylene glycol.

WO 93/10201 discloses a pressure-sensitive poly(N-vinyl lactam) adhesive composition.

EP 0 409 383 discloses estradiol or other estrogen composition for topical application.

EP 0 328 806 describes a transdermal delivery system for estrogen or its derivatives.

WO 2007/016766 discloses a transdermal delivery formulation which includes an organic sulfoxide and a further compound selected from fatty acid ester, fatty acid, azone-related compound and mixtures thereof.

WO 00/44347 relates to a pharmaceutical composition for topical administration in the form of a medicated stick.

The discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention as it existed before the priority date of each claim of this application.

### Summary

The invention provides a transdermal delivery system comprising a composition comprising at least one agent selected from hormones and steroids, a penetration enhancer comprising a polyethylene glycol of average molecular weight no more than 300, and a solvent selected from C₂ to C₄ alkanol and mixtures thereof in an amount in the range of from 70% to 95%, by weight of the total composition.

In a further aspect the invention provides a method of preparing a transdermal delivery system comprising a composition for administration to an area of dermal surface of a subject, the method comprising combining at least one pharmacological agent selected from hormones and steroids, a penetration enhancer comprising polyethylene glycol of average molecular weight no more than 300 and 70% to 95% by weight of the composition of solvent selected from C₂ to C₄ alkanols and mixtures thereof.

The transdermal delivery system will preferably be applied In a dose sufficient to provide an effective amount of at least one pharmacological agent in the bloodstream of an animal.

Preferably the animal is a human but the invention also extends to the treatment of non-human animals.

### Definitions

It will be understood by those skilled in the art that the term polyethylene glycol does not include diethylene glycol (although diethylene glycol may if desired be present as an additional component). Polyethylene glycol of average molecular weight no more than 300 includes polyethylene glycol of nominal average molecular weight 200 and 300 wherein the average molecular weight is not more than 110% and not less than 90% (preferably not more than 105% and not less than 95%) of the nominated value. Polyethylene glycol is of formula H-[OCH₂CH₂]ₙ-OH. An average molecular weight of no more than 300 means the average value of n is at least 3 and is generally from 3 to 6 such as 3, 4, 5 or 6 (although the average need not be an integer) and more preferably 3 to 5. Polyethylene glycol (PEG) is widely available from commercial suppliers in pharmaceutical grades and is sold in specified nominal molecular weights which generally signify that the average molecular weight is not more than 105% and not less than 95% of the nominated value. The viscosities and methods for molecular weight determination are disclosed in USP NF Official Compendium of Standards Volume 11180-1182 [2007 Edition].

The term "pharmacological agent" is used herein to refer to a broad class of useful chemical and therapeutic agents.

The term "pharmacological" in describing the agents contemplated herein is used in a broad sense to comprehend not only agents having a direct pharmacological effect on the host, but also those having an indirect or observable effect which is useful in the medical arts. The term pharmacological agent includes prodrugs of the agent which *in vivo* exerts the physiological effect. Steroids encompass compounds having the general cyclopentanoperhydrophenanthrene ring system of formula:

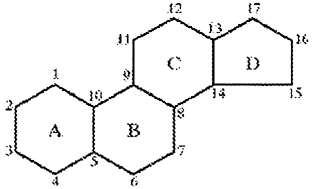

Steroids vary by the functional groups attached to these rings and the oxidation tate of the rings. The steroid may be in the form of the active drug or may be a prodrug steroid which in vivo provides a more active form of the steroid. The steroids include drugs and prodrugs which provide eutrogenic, androgenic glucocorticoid, adrenocortoid, anabolic or birth control activity. Examples of steroids include, for example, dexamethasone, dexamethasone acetate, dexamethasone sodium phosphate, cortisone, cortisone acetate, hydrocortisone, hydrocortisone acetate, hydrocortisone cypionate, hydrocortisone sodium phosphate, hydrocortisone sodium succinate, prednisone, prednisolone, prednisolone acetate, prednisolone sodium phosphate, prednisolone tebutate, prednisolone pivalate, triamcinolone, triamcinolone acetonide, triamcinolone hexacetonide, triamcinolone diacetate, methylprednisolone, methylprednisolone acetate, methylprednisolone sodium succinate, flunsolide, beclomethasone dipropionate, betamethasone sodium phosphate, betamethasone, vetamethasone disodium phosphate, vetamethasone sodium phosphate, betamethasone acetate, betamethasone disodium phosphate, chloroprednisone acetate, corticosterone, desoxycorticosterone, desoxycorticosterone acetate, desoxycorticosterone pivalate, desoximethasone, estradiol, fludrocortisone, fludrocortisone acetate, dichlorisone acetate, fluorohydrocortisone, fluorometholone, fluprednisolone, paramethasone, paramethasone acetate, androsterone, fluoxymesterone, aldosterone, methandrostenolone, methylandrostenediol, methyl testosterone, norethandrolone, testosterone, testosterone enanthate, testosterone propionate, equilenin, equilin, estradiol benzoate, estradiol dipropionate, estriol, estrone, estrone benzoate, acetoxypregnenolone, anagestone acetate, chlormadinone acetate, flurogestone acetate, hydroxymethylprogesterone, hydroxymethylprogesterone acetate, hydroxyprogesterone, hydroxyprogesterone acetate, hydroxyprogesterone caproate, melengestrol acetate, normethisterone, pregnenolone, progesterone, ethynyl estradiol, mestranol, dimethisterone, ethisterone, ethynodiol diacetate, norethindrone, norethindrone acetate, norethisterone, fluocinolone acetonide, flurandrenolone, hydrocortisone sodium succinate, methylprednisolone sodium succinate, prednisolone phosphate sodium, triamcinolone acetonide, hydroxydione sodium, spironolactone, oxandrolone, oxymetholone, prometholone, testosterone cypionate, testosterone phenylacetate, estradiol cypionate, and norethynodrel.

A "prodrug" is a pharmacological drug which is administered in an inactive or less active form and is metabilised into an active form. The prodrug itself may have little or none of the desired activity until it interacts with the systems of the body such as the skin or circulatory systems. Nonetheless hormones and steroids used in the transdermal delivery system of the invention include hormones and steroids which are prodrugs which on administration form a more active hormone or steroid *in vivo* during or after the process of transdermal administration.

In yet another preferred embodiment, a prodrug or a composition of prodrug mixed with the parent composition has a permeation rate that is faster or slower than an identical composition having a pharmacologically equivalent amount of the parent drug. In still another preferred embodiment, the composition has a duration of the therapeutic effect that is longer or shorter than a composition having a pharmacologically equivalent amount of the parent drug alone. In another preferred embodiment, the prodrug is more lipophilic than the parent drug and the prodrug has a greater permeation rate through the skin. Generally the Prohormones and prosteroids are variations or derivatives of the parent hormones or steroids which have groups cleavable under metabolic conditions. Prodrugs become the parent drugs which are pharmaceutically active in vivo, when they undergo solvolysis under physiological conditions or undergo enzymatic degradation. Prodrugs commonly known in the art include acid esters prepared by reaction of the parent acids or alcohol with a suitable alcohol or acid respecctively, or amides prepared by reaction of the parent acid or amine compound with an amine or acid respectively, or basic groups reacted to form an acylated base derivative. Examples of prodrugs are discussed in, Bundgard, Design of Prodrugs, pp. 7-9, 21-24, Elsevier, Amsterdam 1985; Silverman, The Organic Chemistry of Drug Design and Drug Action, pp. 352-401, Academic Press, San Diego, Calif., 1992 and Burger's Medicinal Chemistry and Drug Chemistry, Fifth Ed., Vol. 1, pp. 172-178,949-982 (1995). The other method for controlling the blood plasma profile of subject is in the selection of the prodrug, such as based on its molecular weight or polarity. By increasing the molecular weight of the prodrug, the time to the onset of permeation of effective amounts of the prodrug will increase relatives to the parent drug. One example of this effect is in the use of norethindrone and norethindrone acetate. The permeation rate of norethindrone rapidly peaks after application, whereas norethindrone acetate having a higher molecular weight reaches a maximum after the norethindrone permeation rate begins to decline, steroids having a free hydroxy group at a position on the steroid ring, such as the 17- position, the 3-position, or at the 11-position on the fused ring. Particularly preferred are steroidal hormones such as estrogens, progestins, and androgens. The corresponding steroid prodrug (prosteroid) is defined as a corresponding structure to the steroid where the free hydroxy at the 3,11 or 17 position has been reacted with an alcohol reactive moiety. Particularly preferred are steroid derivatives acylated at the 17 position hydroxyl for example by a C₁-C₁₂ alkanoyl group. Regardless of whether the steroid or the corresponding prosteroid derivative is incorporated in the carrier composition as the dominant drug, each provides a source of steroid in the bloodstream to achieve the intended physiological effect which, in the case of the corresponding prosteroid, occurs through metabolic conversion of the derivative. A steroid ester is the corresponding structure to the steroid where the free hydroxy group on the ring has been esterified. Examples of a steroid and its corresponding ester include estradiol and estradiol benzoate, estradiol 17-beta cypionate, estradiol 17 propionate, estradiol hemisuccinate (eutocol), estradiol enanthate, estradiol undecylate, estradiol acetate, and estradiol proprionate, etc. Another example is testosterone and its corresponding ester of testosterone such as 17 betacypionate, testosterone enanthate, testosterone nicotinate, testosterone phenylacetate, testosterone proprionate, etc. Also included are non-esters that have groups on the 17 position such as testosterone 17-chloral hemiacetal, or ethers that have groups on the 3-position such as estradiol 3-methyl ether.

The terms "percutaneous" and "transdermal" are used herein in the broadest sense to refer to being able to pass through unbroken skin.

The term "dermal penetration enhancer" is used herein in its broadest sense to refer to an agent which improves the rate of percutaneous transport of active agents across the skin for use and delivery of active agents to organisms such as animals, whether it be for local application or systemic delivery.

The term "non-occlusive" is used herein in its broadest sense to refer to not trapping or closing the skin to the atmosphere by means of a patch device, fixed reservoir, application chamber, tape, bandage, sticking plaster, or the like which remains on the skin at the site of application for a prolonged length of time. It is particularly preferred that the transdermal delivery system of the invention is non-occlusive.

The term "stratum corneum" is used herein in its broadest sense to refer to the outer layer of the skin, which is comprised of (approximately 15) layers of terminally differentiated keratinocytes made primarily of the proteinaceous material keratin arranged in a 'brick and mortar' fashion with the mortar being comprised of a lipid matrix made primarily from cholesterol, ceramides and long chain fatty acids. The stratum corneum creates the rate limiting barrier for diffusion of the active agent across the skin.

The term "skin-depot" is used herein in its broadest sense to refer to a reservoir or deposit of active agent and dermal penetration enhancer within the stratum corneum, whether it be intra-cellular (within keratinocytes) or intercellular.

The term "volatile:non-volatile liquid vehicle" is used in the art to refer to a liquid pharmaceutical vehicle comprising a volatile liquid mixed with a non-volatile liquid vehicle, such as a dermal penetration enhancer. A system or vehicle comprising a volatile liquid mixed with a non-volatile dermal penetration enhancer when described herein is used in its broadest sense to include those systems known as volatile: non-volatile liquid vehicles.

The term "aliphatic" includes straight chain, branched chain and cyclic aliphatic and may be saturated alkyl groups or unsaturated aliphatic containing from 1 to 3 unsaturated groups particularly 1 to 3 double bonds.

The transdermal drug delivery system of the present invention enables a wide range of pharmacological agents selected from hormones and steroids to be delivered through the skin to achieve a desired systemic effect. The drug delivery system preferably comprises at least one active agent intimately mixed with a non-volatile dermal penetration enhancer and a volatile liquid. Where the drug delivery system is applied to the skin, at least one active agent selected from hormones and steroids and non-volatile liquid are thermodynamically driven into the skin as the volatile liquid evaporates. Once within the skin the non-volatile liquid may either disrupt the lipid matrix and/or act as a solubilizer to allow an enhanced penetration rate of the at least one active agent through the skin and into the subject being treated. In this way, the dermal penetration enhancer acts as a vehicle and many systemic active agents are able to be transdermally administered to an animal.

The subject to be treated with the transdermal delivery system is generally a mammal, preferably a human being, male or female. The term "therapeutically effective amount" means the amount of the subject compound that will elicit the biological or medical response of a tissue, system, animal or human that is being sought.

Throughout the description and the claims of this specification the word "comprise" and variations of the word, such as "comprising" and "comprises" is not intended to exclude other additives, components, integers or steps.

### Detailed Description

The present inventors have found that the use of polyethylene glycol (of molecular weight no more than 300) as a penetration enhancer shows a significant improvement in penetration enhancement of the active agent.

Typically the PEG of average molecular weight less than 300 will be present in an amount in the range of from 0.5 to 20% such as 1%, 1.5%, 2%, 2.5%, 3%, 3.5%, 4%. 4.5%, 6%, 7%, 8%, 9%, 10%, 11%, 12%, 13%, 14%, 15%, 16%, 17%, 18%, 19%, or 20%. Preferably the PEG of average molecular weight less than 300 is present in an amount in the range of from 0.5 to 15% and most preferably from 0.5 to 10% by weight of the composition.

The composition of the invention preferably comprises PEG 200 in an amount in the range of from 0.1 to 40% by weight of the total composition and preferably from 0.5 to 20% such as 1%, 1.5%, 2%, 2.5%, 3%, 3.5%, 4%, 4.5%, 6%, 7%, 8%, 9%, 10%, 11%, 12%, 13%, 14%, 15%, 16%, 17%, 18%, 19%, 20%.

Preferably the volatile solvent has a vapour pressure above 35 mm Hg at atmospheric pressure and normal skin temperature of 32 degrees Celsius. In a particularly preferred form of the invention the solvent preferably is ethanol or isopropanol, or a mixture thereof.

The volatile solvent is preferably present in the composition of the invention in an amount in the range of 70 to 90% or 75 to 90% by weight of the total composition.

The composition of the invention may if desired contain one or more additional adjuvants such as those selected from the group consisting of penetration enhancers, surfactants, thickeners and solvents. Examples of suitable thickeners include polyacrylic acids; and acylic acid copolymers, agor, carrageenan, food starch, gelatins, germ Arabic, guorgem, hydroxyethyl cellulose hydroxypropymethyl cellulose, protein and polyvinyl pyrrolidone. The content of thickener may be from 0 to 5%.

In one embodiment the penetration enhancer component of the composition may comprise one or more additional penetration enhancers. Of particular note are esters of salicylic acid preferably selected from the Ce to C₃₀ aliphatic ester of salicylic acid and more preferably C₈ to C₁₂ alkyl salicylate and most preferably octyl salicylate particularly 2-ethylhexyl saliclate. When an ester of salicyclic acid is present in combination with polyethylene glycol, the weight ratio of the ester of salicylic add to the polyethylene glycol (of average molecular weight no more than 300) is preferably in the range of from 95:5 to 5:95 and preferably from to 1:10 to 10:1 such as 1:10 to 5:1 and 1:5 to 2:1. The optimal ratio may vary depending on the nature and concentration of the active agent and the concentration of the penetration enhancer combination.

Known dermal penetration enhancers may be included in addition to PEG of molecular weight no more than 300. Examples of known penetration enhancers are laurocapram and laurocapram derivatives, such as those 1-alkylazacycloheptan-2-ones specified in U.S. Pat. No. 5,196,410, and oleic acid and its ester derivatives, such as methyl, ethyl, propyl, isopropyl, butyl, vinyl and glycerylmonooleate, and those given in U.S. Pat. No. 5,082,866, particularly dodecyl (N,N-dimethylamino) acetate and dodecyl (N,N-dimethylamino) propionate and in U.S. Pat. No. 4,861,764, particularly 2-n-nonyl-1-3-dioxolane. Most preferred known dermal penetration enhancers are oleic acid and its ester derivatives, such as methyl, ethyl, propyl, isopropyl, butyl, vinyl and glycerylmonooleate, and those given in U.S. Pat. No. 5,082,866, particulary dodecyl (N,N-dimethylamino) acetate and dodecyl (N,N-dimethylamino) propionate and in U.S. Pat. No. 4,861,764, particularly 2-n-nonyl-1-3-dioxolane.

Preferably the composition will comprise no more than 5% by weight of the other non-volatile penetration enhancer more preferably no more than 1% and most preferably no more than 0.5%by weight of the composition of non-volatile penetration enhancers other than PEG of molecular weight of no more than 300.

In a preferred embodiment of the invention the composition consists essentially of:
(i) at least one active selected from hormones and steroids and more preferably steroidal sex hormones;
(ii) a penetration enhancer component consisting essentially of a polyethylene glycol of average molecular weight no more than 300;
(iii) a volatile solvent consisting of one or more of ethanol and isopropanol;
(iv) optionally a propellant.

It will be understood by those skilled in the art that alcohols and polyols contain a certain amount of water. Typically the total water content of the composition is less than 20% by weight and preferably less than 10% by weight of the total composition.

The composition of the invention may be in a range of forms such as a liquid, cream, paste, gel, lotion, patch (matrix and reservoir), tape, plaster or film former. In the more preferred embodiment the transdermal delivery system is in the form of a liquid for application to a defined area of skin.

The compositions of the present invention may be in any form suitable for topical application to the skin. Suitable forms include sprayable liquids; gels; liquids that may be applied using a roll-on device; lacquers; and sustained release matrices of transdermal delivery devices such as patches. The compositions are usually administered alone but, under some circumstances, administration may be further modified by using other delivery mechanisms such as iontophoresism, ultrasound and microneedles to enhance penetration. Non-occlusive application and in particular spray application is preferred.

Suitable pharmacologically active hormones and steroids may be selected from:

Estrogens such as estradiol, estriol, estradiol benzoate, estradiol 17. beta.-cypionate, estradiol enanthate, estradiol propionate, estrone,ethinylestradiol , Fosfestrol, Dienestrol mestranol, stilboestrol, dienoestrol, epioestriol, estropipate Diethylstilbestrol, Chlorotrianisene, conjugated estrogenic hormones, Polyestradiol phosphate and zeranol and mixtures thereof;

Progesterone and progestins such as norethisterone, norethisterone acetate, gestodene, levonorgestrel, allylestrenol, anagestone, desogestrel, dimethisterone, dydrogesterone, ethisterone, ethynodiol, Ethynodiol diacetate, Etonogestrel, gestodene, ethinylestradiol, haloprogesterone, 17-hydroxy-16-methylene-progesterone, 17. alpha.- hydroxyprogesterone, lynestrenol, medroxyprogesterone, melengestrol, norethindrone, norethynodrel, norgesterone, Gestonorone, Norethisterone, norgestimate, norgestrel, Levonorgestrel, norgestrienone, norvinisterone, pentagestrone, MENT (7-methyl-19-testosterone); Norelgestromin, and trimigestone Drospirenone, Tibolone, and megestrol and mixtures thereof;

Selective progesterone receptor modulators such as Asoprisnil, CDB-4124 and mixtures thereof;

Selective estrogen receptor modulators such as Bazedoxifene, Clomifene, Fulvestrant, Lasofoxifene, Raloxifene, Tamoxifen, Toremifene and mixtures thereof;

Antiprogestogen such as Mifepristone and mixtures thereof.

Antigonadotropins such as Danazol and Gestrinone and mixtures thereof;

Antiandrogens such as cyproterone acetate and danazol and mixtures thereof;

Antiestrogens such as tamoxifen and epitiostanol and the aromatase inhibitors, exemestane and 4-hydroxy-androstenedione and its derivatives and mixtures thereof.

Androgens and anabolic agents such as androisoxazole, androstenediol, bolandiol, bolasterone, clostebol, ethylestrenol. formyldienolone, 4-hydroxy-19-nortestosterone, methandriol, methenolone, methyltrienolone, nandrolone, norbolethone, oxymesterone, stenbolone and trenbolone. Androgenic steroids can include boldenone, fluoxymesterone, mestanolone, mesterolone, methandrostenolone, 17-methyltestosterone, 17. alpha.- methyltestosterone 3-cyclopentyl enol ether, norethandrolone, normethandrone, oxandrolone, oxymesterone, oxymetholone, prasterone, stanlolone, stanozolol, testosterone, testosterone 17-chloral hemiacetal, testosterone proprionate, testosterone enanthate tiomesterone dehydroepiandrosterone (DHEA), androstenedione (Andro): an androstenediol, androsterone, dihydrotestosterone (DHT) and androstanolone and derivatives thereof;

5-alpha reductase inhibitors such as finasteride, turosteride, LY-191704 and MK-306 and mixtures thereof;

Corticosteroids such as betamethasone, betamethasone valerate, cortisone, dexamethasone, dexamethasone 21-phosphate, fludrocortisone, flumethasone, fluocinonide, fluocinonide desonide, fluocinolone, fluocinolone acetonide, fluocortolone, halcinonide, halopredone, hydrocortisone, hydrocortisone 17-valerate, hydrocortisone 17-butyrate, hydrocortisone 21-acetate methylprednisolone, prednisolone, prednisolone 21-phosphate, prednisone, triamcinolone, triamcinolone acetonide and mixtures thereof;

Further examples of steroidal antiinflammatory agents for use in the instant compositions include include cortodoxone, fluoracetonide, fludrocortisone, difluorsone diacetate, flurandrenolone acetonide, medrysone, amcinafel, amcinafide, betamethasone and its other esters, chloroprednisone, clorcortelone, descinolone, desonide, dichlorisone, difluprednate, flucloronide, flumethasone, flunisolide, flucortolone, fluoromethalone, fluperolone, fluprednisolone, meprednisone, methylmeprednisolone, paramethasone, cortisone acetate, hydrocortisone cyclopentylpropionate, cortodoxone, flucetonide, fludrocortisone acetate, flurandrenolone acetonide, medrysone, amcinafal, amcinafide, betamethasone, betamethasone benzoate, chloroprednisone acetate, clocortolone acetate, descinolone acetonide, desoximetasone,dichlorisone acetate, difluprednate, flucloronide, flumethasone pivalate, flunisolide acetate, fluperolone acetate, fluprednisolone valerate,paramethasone acetate, prednisolamate, prednival, triamdnolone hexacetonide, cortivazol, formocortal and nivazol and mixtures thereof;

Aromatase inhibitor such as Aminogluthetimide, Anastrozole, Exemestane, Formestane, Letrozole and Vorozole;

Gonadotropins such as Clomifene and Urofollitropin;

GnRH:(receptor) agonists such as Buserelin, Goserelin, Histrelin, Leuprorelin, Nafarelin and Triptorelin;

GnRH antagonist: Abarelix, Cetrorelix and Ganlrelix;

Pituitary hormones and their active derivatives or analogs such as corticotrophin, thyrotropin, follicle stimulating hormone (FSH), luteinising hormone (LH) and gonadotrophin releasing hormone (GnRH);

Thyroid hormones such as calcitonin, thyroxine and liothyronine and antithyroid agents such as carbimazole and propylthiouracil; and

Other miscellaneous hormone agents such asoctreotide; and mixtures from two or more of the groups.

The optimal ratio of penetration enhancer to active will differ depending on the nature of the active and the penetration enhancer. Typically the weight ratio of penetration enhancer to active will be in the range of from 1000:1 to 1:1000 and preferably from 500:1 to 1:10 and most preferably from 20:1 to 1:1.

The penetration enhancer used according to the invention is particularly useful in transdermal administration of hormones. Hormones that may be used in the drug delivery system of the present invention include systemically active hormones which can be delivered through the skin with the assistance of the dermal penetration enhancer to achieve a desired effect.

Compositions of the invention may include a plurality of hormones from one or more of these groups. For example it may be desirable for contraceptive formulations to comprise one or more estrogens and one or more progestins.

The transdermal delivery system may be used to deliver a therapeutically effective amount of the hormone and/or steroid to a local area or to the systemic circulation. In one embodiment the system provides a pharmaceutically effective level of the pharmacological agent in the systemic circulation, for example a pharmaceutically effective blood level. In one preferred form of the invention the drug delivery system comprises on a weight basis from about 0.1 to about 10% of at least one pharmacological agent selected from hormone and steroids in an amount of from about 0.1 to 12% of the dermal penetration enhancer and from about 78 to 95% ethanol, isopropanol or mixture thereof.

Diseases or conditions that may be treated by using the drug delivery system and methods of the present invention include, but are not limited to, male hormone replacement in testosterone deficient hypogonadal men, female hormone replacement therapy for postmenopausal women using for example estradiol, androgen replacement therapy for females lacking libido using an androgen such as testosterone, male contraception (for example using a progestin such etonogestrel optionally with testosterone) and female contraception (for example using a progestin optionally in combination with an estrogen).

In one embodiment the transdermal delivery system further comprises a spray apparatus comprising a container for a transdermal composition, a spray nozzle and an actuator for delivering a metered dose of spray from the container via the nozzle.

The transdermal delivery system will preferably be applied in a dose sufficient to provide an effective amount of the at least one pharmacological agent in the bloodstream of the animal.

Preferably, the applicator provides a metered dose application such as a metered dose aerosol, a stored-energy metered dose pump or a manual metered dose pump. Preferably the drug delivery system is applied to the skin of the animal covering a delivery surface area between about 10 and 800 cm², more preferably between about 10 and 400 cm², and most preferably between about 10 and 200 cm². The application is most preferably performed by means of a topical metered dose spray combined with an actuator nozzle shroud which together accurately control the amount and/or uniformity of the dose applied. One function of the shroud is to keep the nozzle at a pro-determined height above, and perpendicular to, the skin to which the drug delivery system is being applied. This function may also be achieved by means of a spacer-bar or the like. Another function of the shroud is to enclose the area above the skin in order to prevent or limit bounce-back and/or loss of the drug delivery system to the surrounding environment. Preferably the area of application defined by the shroud is substantially circular in shape.

The invention will now be described with reference to the following examples. It is to be understood that the examples are provided by way of illustration of the invention and that they are in no way limiting to the scope of the invention.

### Examples

The compositions of the Examples and their performance are compared with reference to the drawings.

### Brief Description of Drawings

In the drawings:
Figure 1 is a column chart comparing the permeation of a progestin + an estrogen from a control with progestin transdermal delivery composition of the invention containing PEG-200 pursuant to Example 1.
Figures 2a and 2b are column charts showing the effect on progestin permeation of comparative transdermal compositions containing different progestins and PEG400 rather than PEG 200 as described in Example 2.
Figure 3 is a column chart which shows the effect of PEG 200 on the permeation of an androgen from transdermal delivery compositions described in Example 3.
Figure 4 and Figure 5 are column charts which compare the effect of PEG 200 and PEG 400 respectively on permeation of an androgen from transdermal delivery compositions described in Example 4.
Figure 6 is a column chart examining the effect of PEG 200 on permeation of an androgen from compositions of Example 5.
Figure 7 is a column chart examining the effect of PEG 200 on the permeation of an estrogen from transdermal delivery compositions described in Example 6.
Figure 8 is a column chart showing the effect of PEG 200 on the permeation of the androgen testosterone in the presence of another permeation enhancer as described in Example 7.

### Example 1

### Investigation of the Effect of PEG200 on Cumulative Permeation of the progestin Norethisterone Acetate and the Estrogen Estradiol Through Human Skin In Vitro.

### Methods:

### Finite-dose in vitro diffusion studies were undertaken using dermatomed human female abdominal skin (500 µm).

These experiments were performed over 24 hours using Franz-type cells. Pre-cut skin membranes were mounted as a barrier between the halves of greased (high vacuum grease, BDH) horizontal Franz-type permeation cells in the middle of the receptor chamber of the cell with the stratum corneum facing the donor chamber. The area available for permeation was approximately 0.925 cm². The receptor chambers of the permeation cells were filled with the receptor phase (Phosphate Buffered Saline pH 7.4) and capped. The permeation cells were immersed in a constant temperature water bath such that the receptor chambers were maintained at 35°C. Receptor chamber contents were continuously agitated by small PTFE-coated magnetic stirrer bars driven by submersible magnetic stirrers. The skin was allowed to equilibrate to temperature with receptor solution for 1 h in the water bath prior to dosing.

The formulations were applied to the skin at a dose of 3.6 µL/cm². The applied formulation was spread over the skin area using an Eppendorf positive displacement pipette tip without breaking the skin membrane.

The formulations consisted of:-
○ Comparison composition 1: 2.8% Norethisterone Acetate (NETA), 0.55% Estradiol (E2), 5% Octyl Salicylate (OS)
○ Composition 2: 2.8% NETA, 0.55% E2, 5% Polyethylene Glycol 200 (PEG200)
○ Composition 3: 2.8% NETA, 0.55% E2, 5% OS, 5% PEG200
○ Composition 4: 2.8% NETA, 0.55% E2, 10% PEG200
○ Composition 5: 2.8% NETA, 0.55% E2, 5% OS, 10% PEG200

The amount of active that permeated the skin was quantified using validated HPLC methods

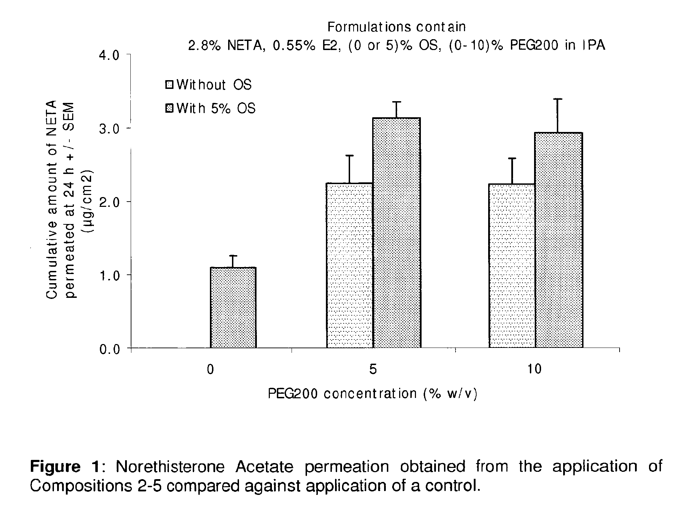

Figure 1 compares the penetration of comparative composition 1 with compositions 2-5 relating to invention. PEG200 in combination with OS was found to significantly enhance the permeation of both Norethisterone Acetate and estradiol through human epidermis *in vitro.* Permeation of NETA is compared in Figure 1.

### Example 2

### Investigation Into the Effect of PEG200 and PEG400 on Cumulative Nestorone & Ethinylestradiol Permeation Through Human Skin In Vitro

### Methods:

Finite-dose *in vitro* diffusion studies were undertaken using dermatomed human female abdominal skin(500 µm).

These experiments were performed over 24 hours using stainless steel, flow through diffusion cells based on those described previously (Cooper, E.R. J. Pharm. Sci. 1984, 73, 1153-1156) except that the cell was modified to increase the diffusion area to 1.0cm². The formulations were applied using a finite dose technique (Franz, T.J. Curr. Probl. Dermatol., 1978, 7, 58-68) to mimic clinical dosing conditions at an applied dose volume of 3.6 µL /cm². A piece of stainless steel wire mesh was placed directly below the skin in the receptor chamber of the of the diffusion cell to maintain a turbulent flow of receptor solution below the skin. The diffusion cells were maintained at a flow rate of approximately 0.5 mL/hr by a microcassette peristaltic pump (Watson Marlow 505S UK). The cells were kept at 32 ± 0.5°C by a heater bar and the samples were collected into appropriately sized glass vials for a period of 24 hr. The receptor solutions (Phosphate Buffered Saline pH7.4) maintained sink conditions below the skin.

The formulations consisted of:-
○ Composition (Comp) 1 (Control): 1.35% Nestorone (NES), 0.35% Ethinylestradiol (EE) in Isopropyl Alcohol (IPA)
○ Comp 2: 1.35% NES, 0.35% EE, 5% Polyethylene glycol 400 (PEG400) in IPA Comp 3: 1.35% NES, 0.35% EE, 0.5% Polyethylene glycol (PEG200) in IPA

The amount of active that permeated the skin was quantified using validated HPLC methods.

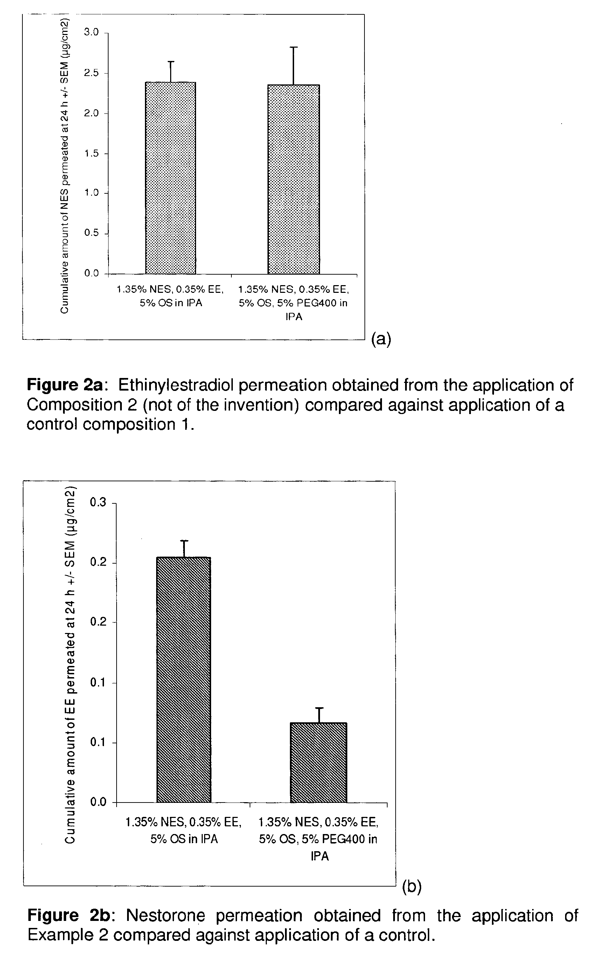

The effect of PEG400 on permeation of NES and EE is shown in Figures 2a and 2b respectively. **Figures 2a and 2b****:** thus show Nestorone and Ethinylestradiol permeation respectively obtained from the application of Composition 2 (not of the invention) compared against application of a control composition 1.

PEG200 in combination with OS was found to enhance the permeation of both Nestorone and Ethinylestradiol through human epidermis *in vitro.*

The addition of PEG400 to the formulation did not have a significant effect (enhancing or inhibitory) on the permeation of Nestorone through human epidermis *in vitro.* PEG400 was found to inhibit the permeation of ethinylestradiol through human epidermis *in vitro.*

### Example 3

### Investigation Into the Effect of PEG200 on Cumulative Testosterone Permeation Through Human Skin In Vitro

### Methods:

Finite-dose *in vitro* diffusion studies were undertaken using dermatomed human female abdominal skin (500 µm).

These experiments were performed over 24 hours using stainless steel, flow through diffusion cells based on those described previously (Cooper, E.R. J. Pharm. Sci. 1984, 73, 1153-1156) except that the cell was modified to increase the diffusion area to 1.0cm². The formulations were applied using a finite dose technique (Franz, T.J. Curr. Probl. Dermatol., 1978, 7, 58-68) to mimic clinical dosing conditions at an applied dose volume of 3.6 µL /cm². A piece of stainless steel wire mesh was placed directly below the skin in the receptor chamber of the of the diffusion cell to maintain a turbulent flow of receptor solution below the skin. The diffusion cells were maintained at a flow rate of approximately 1.0 mL/hr by a microcassette peristaltic pump (Watson Marlow 505S UK). The cells were kept at 32 ± 0.5°C by a heater bar and the samples were collected into appropriately sized glass vials for a period of 24 hr. The receptor solutions (0.002%w/v NaN₃) maintained sink conditions below the skin.

The formulations consisted of:-
○ Comp 1: 5% Testosterone (TES), in ethanol (95%)
○ Comp 2: 5% TES, 1.0% polyethylene glycol 200 (PEG200) in ethanol (95%)
○ Comp 3: 5% TES, 2.5% PEG200 in ethanol (95%)

The amount of active that permeated the skin was quantified using validated HPLC methods.

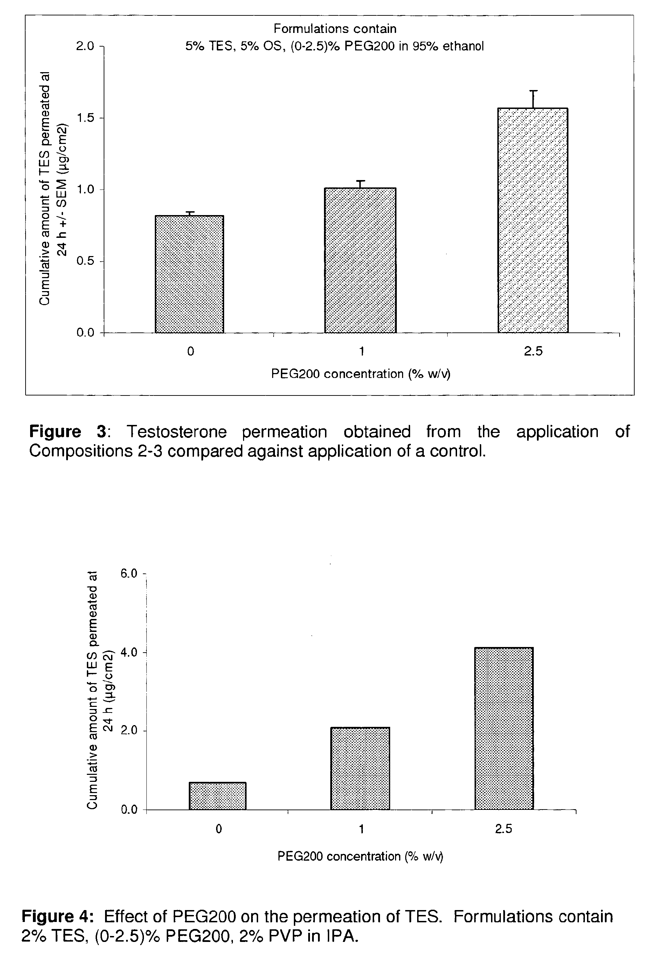

The effect of the combination of PEG200 in Compositions 2 and 3 is compared with a control Composition 1 in Figure 3 As shown in Figure 3, PEG200 in was found to significantly enhance the permeation of Testosterone through human epidermis *in vitro.*

### Example 4

### Investigation into the Effect of PEG200 and PEG 400 on Cumulative Permeation of the AndrogenTestosterone from a lotion through Human Skin In Vitro.

### Methods:

Finite-dose *in vitro* permeation studies were undertaken using dermatomed skin (Padgett Model B or S electric dermatome set at 500 µm) prepared from excised female, abdominal tissue.

These experiments were conducted over 24 hours (h) using flow-through systems with a 1-cm² administration area. A piece of stainless steel wire mesh was placed in the receptor chamber of each permeation cell to support the skin and to maintain a turbulent flow of receptor solution below the skin. The receptor solution was maintained at a flow rate of approximately 0.5 mL/hr by a peristaltic pump (Watson Marlow 520S Peristaltic Pump with 313A adaptor and 308MC 8 roller pump-head; Stauff Corporation, Australia). The cells were placed on a heater bar to keep the temperature of the skin at 32 ± 1°C.

Following a 2-h equilibration of the skin with the receptor solution (RS; 0.002% sodium azide), the stratum corneum surface was dosed with either 15 or 30 µL/cm² of an MD-Lotion formulation using a positive displacement pipette. The formulation was spread evenly over the skin area using the pipette tip. Permeation samples were collected into appropriately sized glass vials for a period of 24 h.

The effect of the addition of Polyethylene glycol 200 (PEG200) or Polyethylene glycol 400 (PEG 400) on the permeation of testosterone was investigated. 0.5-5%w/v PEG200 or PEG 400 was added to the following Testosterone (TES) Lotion formulation:-• Formulation: 2%w/v TES, 2%w/v polyvinylpyrrolidone (PVP) in isopropyl alcohol (IPA).

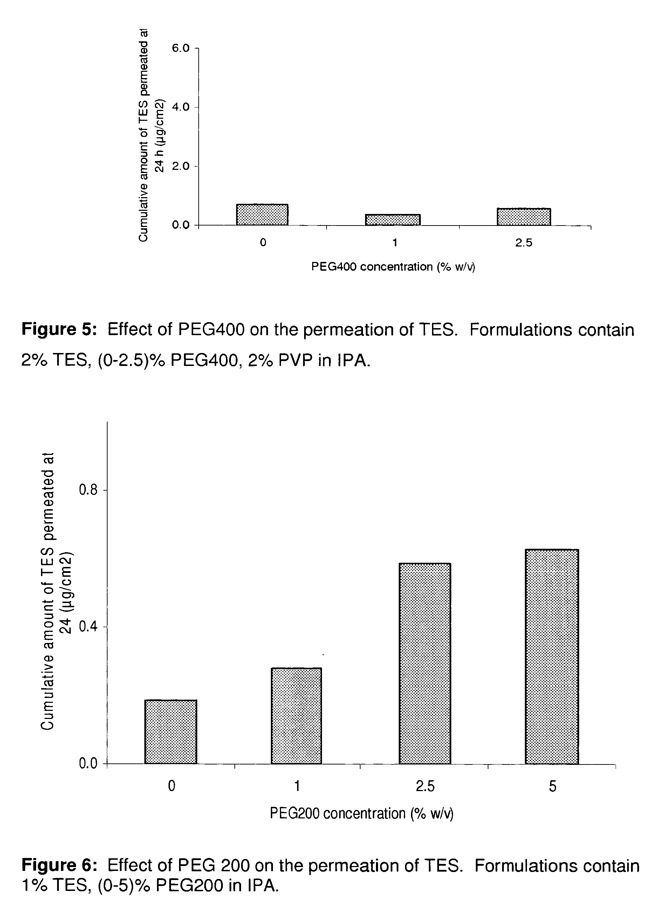

The amount of active that permeated the skin was quantified using validated HPLC methods. Results for PEG 200 are shown in Figure 4 and results for PEG 400 are shown in Figure 5.

### Results:

PEG200 significantly enhanced the permeation of TES through human skin *in vitro.* PEG400 did not have any effect on the permeation of TES through human skin *in vitro.*

### Example 5

### Investigation Into the Effect of PEG200 and PEG 400 on Cumulative Permeation of the Androgen Testosterone Through Human Skin From a Transdermal Testosterone Composition Applied as a Spray In Vitro

### Methods:

Finite-dose *in vitro* permeation studies were undertaken using dermatomed skin (Padgett Model B or S electric dermatome set at 500 µm) prepared from excised female, abdominal skin.

These experiments were conducted over 24 hours (h) using flow-through systems with a 1-cm² administration area. A piece of stainless steel wire mesh was placed in the receptor chamber of the of each permeation cell to support the skin and to maintain a turbulent flow of receptor solution below the skin. The receptor solution was maintained at a nominal flow rate of 0.5 mL/h by a peristaltic pump (Watson Marlow 520S Peristaltic Pump with 313A adaptor and 308MC 8 roller pump-head; Stauff Corporation, Australia). The cells were placed on a heater bar to keep the temperature of the skin at 32 ± 1°C.

Following a 2-h equilibration of the skin with the receptor solution (RS; 0.002% sodium azide), the stratum corneum surface was dosed with 3.6 µL/cm² of a Metered Dose Transdermal Spray (MDTS) formulation using a positive displacement pipette. The formulation was spread evenly over the skin area using the pipette tip. Permeation samples were collected into appropriately sized glass vials for a period of 24 h.

The Metered Dose Transdermal Spray formulations contained:
**•** Testosterone (TES), Polyethylene glycol 200 (PEG 200) or Polyethylene glycol 400 (PEG 400) isopropyl alcohol (IPA).

The amount of active that permeated the skin was quantified using validated HPLC methods and the results for PEG 200 are shown in Figure 6.

### Results:

PEG200 increased the permeation of TES through human skin *in vitro.* The addition of Adding PEG 400 to the formulation did not result in any significant difference in the permeation of TES when compared with the control formulation.

### Example 6

### Estradiol Spray: Investigation Into the Effect of PEG200 and PEG 400 on Estradiol Permeation Through Human Skin In Vitro

### Methods:

Finite-dose *in vitro* permeation studies were undertaken using dermatomed skin (Padgett Model B or S electric dermatome set at 500 µm) prepared from excised female, abdominal skin.

These experiments were conducted over 24 hours (h) using flow-through systems with a 1-cm² administration area. A piece of stainless steel wire mesh was placed in the receptor chamber of the of each permeation cell to support the skin and to maintain a turbulent flow of receptor solution below the skin. The receptor solution was maintained at a nominal flow rate of 0.5 mL/h by a peristaltic pump (Watson Marlow 520S Peristaltic Pump with 313A adaptor and 308MC 8 roller pump-head; Stauff Corporation, Australia). The cells were placed on a heater bar to keep the temperature of the skin at 32 ± 1°C.

Following a 2-h equilibration of the skin with the receptor solution (RS; 0.002% sodium azide), the stratum corneum surface was dosed with 3.6 µL/cm² of an Estradiol Transdermal Spray formulation using a positive displacement pipette. The formulation was spread evenly over the skin area using the pipette tip. Permeation samples were collected into appropriately sized glass vials for a period of 24 h.

The Estradiol Transdermal Spray formulations contained:-
• Testosterone (TES), Polyethylene glycol 200 (PEG 200) or Polyethylene glycol 400 (PEG 400) isopropyl alcohol (IPA)

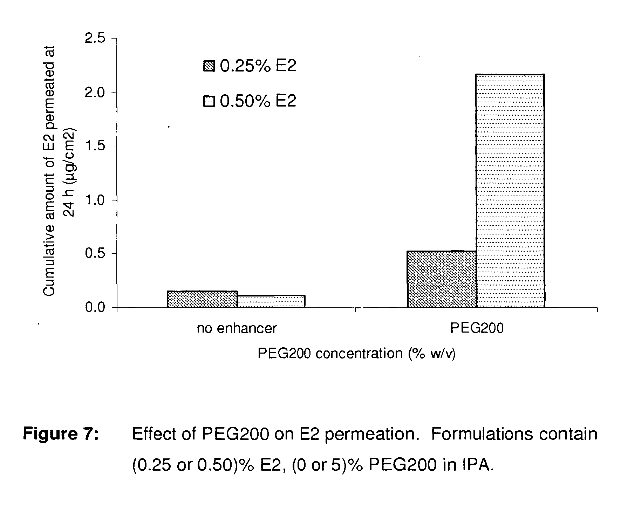

The amount of active that permeated the skin was quantified using validated HPLC methods and the results are depicted in Figure 7..

### Results:

In both the 0.25% and the 0.50% formulations PEG200 was found to enhance the permeation of Estradiol through human skin *in vitro.*

### Example 7

### Investigation Into the Effect of PEG200 on Cumulative Testosterone Permeation Through Human Skin In Vitro

### Methods:

Finite-dose *in vitro* diffusion studies were undertaken using dermatomed human female abdominal skin (500 µm).

These experiments were performed over 24 hours using stainless steel, flow through diffusion cells based on those described previously (Cooper, E.R. J. Pharm. Sci. 1984, 73, 1153-1156) except that the cell was modified to increase the diffusion area to 1.0cm². The formulations were applied using a finite dose technique (Franz, T.J. Curr. Probl. Dermatol., 1978, 7, 58-68) to mimic clinical dosing conditions at an applied dose volume of 15 µL /cm². A piece of stainless steel wire mesh was placed directly below the skin in the receptor chamber of the of the diffusion cell to maintain a turbulent flow of receptor solution below the skin. The diffusion cells were maintained at a flow rate of approximately 1.0 mL/hr by a microcassette peristaltic pump (Watson Marlow 505S UK). The cells were kept at 32 ± 0.5°C by a heater bar and the samples were collected into appropriately sized glass vials for a period of 24 hr. The receptor solutions (0.002%w/v NaN₃) maintained sink conditions below the skin.

The formulations consisted of:-
○ Comp 1: 2% Testosterone (TES), 5% Octyl Salicylate (OS), 2% polyvinyl pyrrolidine (PVP), 30% isopropyl alcohol (IPA) in ethanol (95%)
○ Comp 2: 2% TES, 5% OS, 2% PVP, 30% IPA, 0.5% polyethylene glycol 200 (PEG200) in ethanol (95%)
○ Comp 3: 2% TES, 5% OS, 2% PVP, 30% IPA, 1.0% PEG200 in ethanol (95%)
○ Comp 4: 2% TES, 5% OS, 2% PVP, 30% IPA, 2.5% PEG200 in ethanol (95%)

The amount of active that permeated the skin was quantified using validated HPLC methods

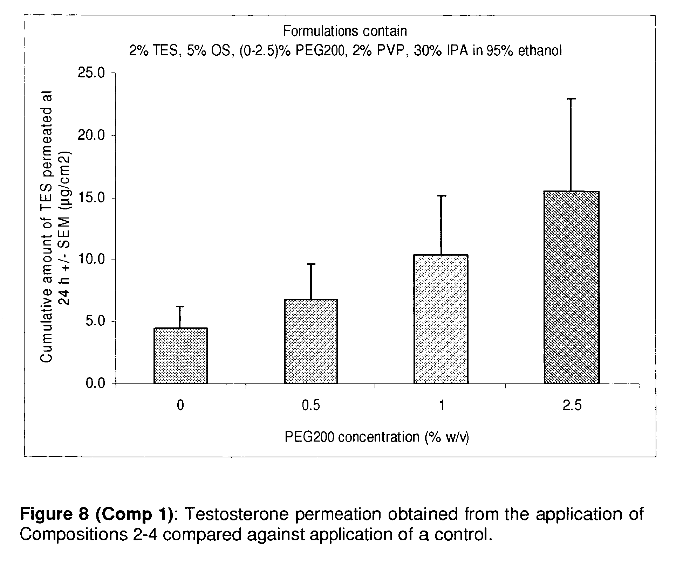

The effect on permeation of TES from using the composition as shown in Figure 8. PEG200 was found to significantly enhance the permeation of Testosterone in combination with the permeation enhancer octyl salicylate (OS) through human epidermis *in vitro.*

## Claims

1. A transdermal delivery system comprising a composition comprising at least one pharmacological agent selected from hormones and steroids; a penetration enhancer comprising a polyethylene glycol (PEG) of average molecular weight no more than 300; and a solvent selected from C₂ to C₄ alkanol and mixtures thereof in an amount in the range of from 70% to 95%, by weight of the total composition.

2. A transdermal delivery system according to claim 1 wherein the PEG of average molecular weight of no more than 300 is present in an amount of at least 0.1% by weight of the total composition.

3. A transdermal delivery system according to any one of the previous claims wherein the polyethylene glycol of average weight no more than 300 is present in an amount in the range of from 0.5% to 20% by weight of the total composition.

4. A transdermal delivery system according to any one of the previous claims wherein the composition consists essentially of:
(i) the at least one pharmacological agent selected from hormones and steroids;
(ii) the penetration enhancer component consisting of a polyethylene glycol of average molecular weight no more than 300;
(iii) a volatile solvent consisting of one or more of ethanol and isopropanol; and
(iv) optionally a propellant.

5. A transdermal delivery system according to any one of the previous claims wherein the total water content of the composition is less than 10% by weight of the total composition.

6. A transdermal delivery system according to any one of the previous claims which is non-occlusive.

7. A transdermal delivery system according to any one of the previous claims wherein the weight ratio of penetration enhancer to pharmacological agent is in the range of from 20:1 to 1:1.

8. A transdermal delivery system according to any one of the previous claims wherein at least one pharmacological agent comprises one or more selected from the group consisting of steroidal hormones.

9. A transdermal delivery system according to any one of the previous claims wherein at least one pharmacological agent comprises one or more steroids which provide eutrogenic, androgenic, glucocorticoid, adrenocortoid, anabolic or birth control activity.

10. A transdermal delivery system according to claim 9 wherein the pharmacological agent comprises one or more steroids selected from the group consisting of dexamethasone, dexamethasone acetate, dexamethasone sodium phosphate, cortisone, cortisone acetate, hydrocortisone, hydrocortisone acetate, hydrocortisone cypionate, hydrocortisone sodium phosphate, hydrocortisone sodium succinate, prednisone, prednisolone, prednisolone acetate, prednisolone sodium phosphate, prednisolone tebutate, prednisolone pivalate, triamcinolone, triamcinolone acetonide, triamcinolone hexacetonide, triamcinolone diacetate, methylprednisolone, methylprednisolone acetate, methylprednisolone sodium succinate, flunsolide, beclomethasone dipropionate, betamethasone sodium phosphate, betamethasone, vetamethasone disodium phosphate, vetamethasone sodium phosphate, betamethasone acetate, betamethasone disodium phosphate, chloroprednisone acetate, corticosterone, desoxycorticosterone, desoxycorticosterone acetate, desoxycorticosterone pivalate, desoximethasone, estradiol, fludrocortisone, fludrocortisone acetate, dichlorisone acetate, fluorohydrocortisone, fluorometholone, fluprednisolone, paramethasone, paramethasone acetate, androsterone, fluoxymesterone, aldosterone, methandrostenolone, methylandrostenediol, methyl testosterone, norethandrolone, testosterone, testosterone enanthate, testosterone propionate, equilenin, equilin, estradiol benzoate, estradiol dipropionate, estriol, estrone, estrone benzoate, acetoxypregnenolone, anagestone acetate, chlormadinone acetate, flurogestone acetate, hydroxymethylprogesterone, hydroxymethylprogesterone acetate, hydroxyprogesterone, hydroxyprogesterone acetate, hydroxyprogesterone caproate, melengestrol acetate, normethisterone, pregnenolone, progesterone, ethynyl estradiol, mestranol, dimethisterone, ethisterone, ethynodiol diacetate, norethindrone, norethindrone acetate, norethisterone, fluocinolone acetonide, flurandrenolone, hydrocortisone sodium succinate, methylprednisolone sodium succinate, prednisolone phosphate sodium, triamcinolone acetonide, hydroxydione sodium, spironolactone, oxandrolone, oxymetholone, prometholone, testosterone cypionate, testosterone phenylacetate, estradiol cypionate, and norethynodrel and the salts and prodrugs thereof.

11. A transdermal delivery system according to any one of the previous claims for female contraception comprising one or more estrogens and one or more progestins.

12. A transdermal delivery system according to any one of the previous claims wherein the drug delivery system comprises on a weight basis from about 0.1 to about 10% of the steroid or hormone, from about 0.1 to 12% of the penetration enhancer and from about 70 to 95% ethanol, isopropanol or mixture thereof.

13. A transdermal delivery system according to claim 1 further comprising a spray apparatus comprising a container containing the transdermal composition, a spray nozzle and an actuator for delivering a metered dose of spray from the container via the spray nozzle.

14. A transdermal delivery system according to any one of claims 1 to 13 for transdermal administration of at least one pharmacological agent selected from hormones and steroids.

15. The transdermal delivery system according to claim 14, wherein the transdermal administration is by application of the medicament to an area of dermal surface of a subject.

16. The transdermal delivery system according to claim 15 wherein the subject is in need of male hormone replacement in testosterone deficient hypogonadal men, female hormone replacement therapy for postmenopausal women, or androgen replacement therapy for females lacking libido or suffering depression using an androgen, male contraception or female contraception.

17. A method of preparing a transdermal delivery system comprising a composition for administration to an area of dermal surface of a subject, the method comprising combining at least one pharmacological agent selected from hormones and steroids, a penetration enhancer comprising polyethylene glycol of average molecular weight no more than 300 and 70% to 95% by weight of the composition of solvent selected from C₂ to C₄ alkanols and mixtures thereof.

18. A transdermal delivery system according to claim 1 wherein the PEG of average molecular weight no more than 300 is PEG 200 in an amount of from 0.1 to 40% by weight of the total composition.

19. A transdermal delivery system according to claim 1 wherein the PEG of average molecular weight no more than 300 is of formula H-[OCH₂ CH₂)ₙ-OH where n is 4.

## Patentansprüche

1. Transdermales Zuführsystem, das eine Zusammensetzung umfasst, die mindestens ein pharmakologisches Mittel, das aus Hormonen und Steroiden ausgewählt ist, einen Penetrationsverstärker, der ein Polyethylenglykol (PEG) eines durchschnittlichen Molekulargewichts von nicht mehr als 300 umfasst, und ein Lösungsmittel, das aus C₂ bis C₄-Alkanol und Gemischen davon ausgewählt ist, in einer Menge von 70% bis 95% nach Gewicht der Gesamtzusammensetzung umfasst.

2. Transdermales Zuführsystem gemäß Anspruch 1, wobei das PEG eines durchschnittlichen Molekulargewichts von nicht mehr als 300 in einer Menge von mindestens 0,1% nach Gewicht der Gesamtzusammensetzung vorliegt.

3. Transdermales Zuführsystem gemäß einem der vorhergehenden Ansprüche, wobei das Polyethylenglykol eines durchschnittlichen Molekulargewichts von nicht mehr als 300 in einer Menge von 0,5% bis 20% nach Gewicht der Gesamtzusammensetzung vorliegt.

4. Transdermales Zuführsystem gemäß einem der vorhergehenden Ansprüche, wobei die Zusammensetzung im Wesentlichen besteht aus:
(i) dem mindestens einen pharmakologischen Mittel, das aus Hormonen und Steroiden ausgewählt ist,
(ii) der Penetrationsverstärkerkomponente, die aus einem Polyethylenglykol eines durchschnittlichen Molekulargewichts von nicht mehr als 300 besteht,
(iii) einem flüchtigen Lösungsmittel, das aus einem oder mehreren von Ethanol und Isopropanol besteht, und
(iv) gegebenenfalls einem Treibmittel.

5. Transdermales Zuführsystem gemäß einem der vorhergehenden Ansprüche, wobei der gesamte Wassergehalt der Zusammensetzung weniger als 10% nach Gewicht der Gesamtzusammensetzung beträgt.

6. Transdermales Zuführsystem gemäß einem der vorhergehenden Ansprüche, das nicht-okklusiv ist.

7. Transdermales Zuführsystem gemäß einem der vorhergehenden Ansprüche, wobei das Gewichtsverhältnis von Penetrationsverstärker zu pharmakologischem Mittel 20:1 bis 1:1 beträgt.

8. Transdermales Zuführsystem gemäß einem der vorhergehenden Ansprüche, wobei mindestens ein pharmakologisches Mittel eines oder mehreres aus der Gruppe, bestehend aus Steroidhormonen, umfasst.

9. Transdermales Zuführsystem gemäß einem der vorhergehenden Ansprüche, wobei mindestens ein pharmakologisches Mittel ein oder mehrere Steroide umfasst, die eutrogene, androgene, glukokortikale, adrenokortikale, anabole Aktivität oder Geburtskontrollaktivität bereitstellen.

10. Transdermales Zuführsystem gemäß Anspruch 9, wobei das pharmakologische Mittel ein oder mehrere Steroide umfasst, die ausgewählt sind aus der Gruppe, bestehend aus Dexamethason, Dexamethasonacetat, Dexamethasonnatriumphosphat, Cortison, Cortisonacetat, Hydrocortison, Hydrocortisonacetat, Hydrocortisoncypionat, Hydrocortisonnatriumphosphat, Hydrocortisonnatriumsuccinat, Prednison, Prednisolon, Prednisolonacetat, Prednisolonnatriumphosphat, Prednisolontebutat, Prednisolonpivalat, Triamcinolon, Triamcinolonacetonid, Triamcinolonhexacetonid, Triamcinolondiacetat, Methylprednisolon, Methylprednisolonacetat, Methylprednisolonnatriumsuccinat, Flunsolid, Beclomethasondipropionat, Betamethasonnatriumphosphat, Betamethason, Vetamethasondinatriumphosphat, Vetamethasonnatriumphosphat, Betamethasonacetat, Betamethasondinatriumphosphat, Chlorprednisonacetat, Corticosteron, Desoxycorticosteron, Desoxycorticosteronacetat, Desoxycorticosteronpivalat, Desoximethason, Estradiol, Fludrocortison, Fludrocortisonacetat, Dichlorisonacetat, Fluorhydrocortison, Fluormetholon, Fluprednisolon, Paramethason, Paramethasonacetat, Androsteron, Fluoxymesteron, Aldosteron, Methandrostenolon, Methylandrostendiol, Methyltestosteron, Norethandrolon, Testosteron, Testosteronenanthat, Testosteronpropionat, Equilenin, Equilin, Estradiolbenzoat, Estradioldiproprionat, Estriol, Estron, Estronbenzoat, Acetoxypregnenolon, Anagestonacetat, Chlormadinonacetat, Flurogestonacetat, Hydroxymethylprogesteron, Hydroxymethylprogesteronacetat, Hydroxyprogesteron, Hydroxyprogesteronacetat, Hydroxyprogesteroncaproat, Melengestrolacetat, Normethisteron, Pregnenolon, Progesteron, Ethinylestradiol, Mestranol, Dimethisteron, Ethisteron, Ethinodioldiacetat, Norethindron, Norethindronacetat, Norethisteron, Fluocinolonacetonid, Flurandrenolon, Hydrocortisonnatriumsuccinat, Methylprednisolonnatriumsuccinat, Prednisolonphosphatnatrium, Triamcinolonacetonid, Hydroxydionnatrium, Spironolacton, Oxandrolon, Oxymetholon, Prometholon, Testosteroncypionat, Testosteronphenylacetat, Estradiolcypionat und Norethinodrel und den Salzen und Arzneimittelvorläufern davon.

11. Transdermales Zuführsystem gemäß einem der vorhergehenden Ansprüche für eine weibliche Empfängnisverhütung, das ein oder mehrere Estrogene und ein oder mehrere Progestine umfasst.

12. Transdermales Zuführsystem gemäß einem der vorhergehenden Ansprüche, wobei das Arzneimittelzuführsystem auf einer Gewichtsbasis etwa 0,1 bis etwa 10% des Steroids oder Hormons, etwa 0,1 bis 12% des Penetrationsverstärkers und etwa 70 bis 95% Ethanol, Isopropanol oder ein Gemisch davon umfasst.

13. Transdermales Zuführsystem gemäß Anspruch 1, das ferner eine Sprühvorrichtung umfasst, die einen Behälter, der die transdermale Zusammensetzung enthält, eine Sprühdüse und ein Bedienelement zum Zuführen einer gemessenen Dosis des Sprays von dem Behälter über die Sprühdüse umfasst.

14. Transdermales Zuführsystem gemäß einem der Ansprüche 1 bis 13 für eine transdermale Verabreichung mindestens eines pharmakologischen Mittels, das aus Hormonen und Steroiden ausgewählt ist.

15. Transdermales Zuführsystem gemäß Anspruch 14, wobei die transdermale Verabreichung durch Applizieren des Medikaments an einen Hautoberflächenbereich eines Lebewesens erfolgt.

16. Transdermales Zuführsystem gemäß Anspruch 15, wobei das Lebewesen einen Bedarf für einen männlichen Hormonaustausch in testosterondefizitären hypogonadalen Männern, eine weibliche Hormonaustauschtherapie für postmenopausale Frauen oder eine Androgenaustauschtherapie für Frauen, denen Libido fehlt oder die an Depression leiden, unter Verwendung eines Androgens, männliche Empfängnisverhütung oder weibliche Empfängnisverhütung hat.

17. Verfahren zum Herstellen eines transdermalen Zuführsystems, das eine Zusammensetzung für eine Verabreichung an einen Hautoberflächenbereich eines Lebewesens umfasst, wobei das Verfahren ein Vereinigen von mindestens einem pharmakologischen Mittel, das aus Hormonen und Steroiden ausgewählt ist, eines Penetrationsverstärkers, der Polyethylenglykol eines durchschnittlichen Molekulargewichts von nicht mehr als 300 umfasst, und 70% bis 95% nach Gewicht der Zusammensetzung eines Lösungsmittels, dass aus C₂ bis C₄-Alkanolen und Gemischen davon ausgewählt ist, umfasst.

18. Transdermales Zuführsystem gemäß Anspruch 1, wobei das PEG eines durchschnittlichen Molekulargewicht von nicht mehr als 300 PEG 200 in einer Menge von 0,1 bis 40% nach Gewicht der Gesamtzusammensetzung ist.

19. Transdermales Zuführsystem gemäß Anspruch 1, wobei das PEG eines durchschnittlichen Molekulargewichts von nicht mehr als 300 der Formel H-[OCH2 CH₂]ₙ-OH entspricht, wobei n 4 beträgt.

## Revendications

1. Système de délivrance transdermique comprenant une composition comprenant au moins un agent pharmacologique choisi parmi les hormones et les stéroïdes; un renforçateur de pénétration comprenant un polyéthylène glycol (PEG) ayant une masse moléculaire moyenne ne dépassant pas 300; et un solvant choisi parmi un alcool en C₂ à C₄ et leurs mélanges en une quantité dans l'intervalle de 70% à 95%, en poids de la composition totale.

2. Système de délivrance transdermique selon la revendication 1, dans lequel le PEG ayant une masse moléculaire moyenne ne dépassant pas 300 est présent en une quantité d'au moins 0,1 % en poids de la composition totale.

3. Système de délivrance transdermique selon l'une quelconque des revendications précédentes, dans lequel le polyéthylène glycol ayant une masse moléculaire moyenne ne dépassant pas 300 est présent en une quantité dans l'intervalle de 0,5% à 20% en poids de la composition totale.

4. Système de délivrance transdermique selon l'une quelconque des revendications précédentes, dans lequel la composition consiste essentiellement en:
(i) le au moins un agent pharmacologique choisi parmi les hormones et les stéroïdes;
(ii) le composant renforçateur de pénétration consistant en un polyéthylène glycol ayant une masse moléculaire moyenne ne dépassant pas 300;
(iii) un solvant volatil consistant en un ou plusieurs parmi l'éthanol et l'isopropanol; et
(iv) en option un agent propulseur.

5. Système de délivrance transdermique selon l'une quelconque des revendications précédentes, dans lequel la teneur totale en eau de la composition est inférieure à 10% en poids de la composition totale.

6. Système de délivrance transdermique selon l'une quelconque des revendications précédentes, qui est non-occlusif.

7. Système de délivrance transdermique selon l'une quelconque des revendications précédentes, dans lequel le rapport en poids du renforçateur de pénétration à l'agent pharmacologique est dans l'intervalle de 20:1 à 1:1.

8. Système de délivrance transdermique selon l'une quelconque des revendications précédentes, dans lequel au moins un agent pharmacologique comprend un ou plusieurs choisis dans le groupe consistant en hormones stéroïdiennes.

9. Système de délivrance transdermique selon l'une quelconque des revendications précédentes, dans lequel au moins un agent pharmacologique comprend un ou plusieurs stéroïdes qui procurent une activité eutrogénique, androgénique, de glucocorticoïde, d'adrénocorticoïde, anabolisante ou de régulation des naissances.

10. Système de délivrance transdermique selon la revendication 9, dans lequel l'agent pharmacologique comprend un ou plusieurs stéroïdes choisis dans le groupe consistant en dexaméthasone, acétate de dexaméthasone, phosphate sodique de dexaméthasone, cortisone, acétate de cortisone, hydrocortisone, acétate d'hydrocortisone, cypionate d'hydrocortisone, phosphate sodique d'hydrocortisone, succinate sodique d'hydrocortisone, prednisone, prednisolone, acétate de prednisolone, phosphate sodique de prednisolone, tébutate de prednisolone, pivalate de predsinolone, triamcinolone, acétonide de triamcinolone, hexacétonide de triamcinolone, diacétate de triamcinolone, méthylprednisolone, acétate de méthylprednisolone, succinate sodique de méthylprednisolone, flunsolide, dipropionate de béclométhasone, phosphate sodique de bétaméthasone, bétaméthasone, phosphate disodique de vétaméthasone, phosphate sodique de vétaméthasone, acétate de bétaméthasone, phosphate disodique de bétaméthasone, acétate de chloroprednisone, corticostérone, désoxycorticostérone, acétate de désoxycorticostérone, pivalate de désoxycorticostérone, désoxyméthasone, oestradiol, fludrocortisone, acétate de fludrocortisone, acétate de dichlorisone, fluorohydrocortisone, fluorométholone, fluprednisolone, paraméthasone, acétate de paraméthasone, androstérone, fluoxymestérone, aldostérone, méthandrosténolone, méthylandrostènediol, méthyl testostérone, noréthandrolone, testostérone, enanthate de testostérone, propionate de testostérone, équilénine, équiline, benzoate d'oestradiol, dipropionate d'oestradiol, oestriol, oestrone, benzoate d'oestrone, acétoxypregnénolone, acétate d'anagestone, acétate de chlormadinone, acétate de flurogestone, hydroxyméthylprogestérone, acétate d' hydroxyméthylprogestérone, hydroxyprogestérone, acétate d'hydroxyprogestérone, caproate d'hydroxyprogestérone, acétate de mélengestrol, norméthistérone, pregnénolone, progestérone, éthynyl oestradiol, mestranol, diméthistérone, éthistérone, diacétate d'éthynodiol, noréthindrone, acétate de noréthindrone, norethistérone, acétonide de fluocinolone, flurandrénolone, succinate sodique d'hydrocortisone, succinate sodique de méthylprednisolone, phosphate sodique de prednisolone, acétonide de triamcinolone, hydroxydione sodique, spironolactone, oxandrolone, oxymétholone, prométholone, cypionate de testostérone, phénylacétate de testostérone, cypionate d'oestradiol, et noréthynodrel, et leurs sels et promédicaments.

11. Système de délivrance transdermique selon l'une quelconque des revendications précédentes pour contraception féminine, comprenant un ou plusieurs oestrogènes et un ou plusieurs progestatifs.

12. Système de délivrance transdermique selon l'une quelconque des revendications précédentes, dans lequel le système de délivrance de médicament comprend sur une base pondérale d'environ 0,1 à environ 10% du stéroïde ou de l'hormone, d'environ 0,1 à 12% du renforçateur de pénétration et d'environ 70 à 95% d'éthanol, d'isopropanol ou de leur mélange.

13. Système de délivrance transdermique selon la revendication 1, comprenant en outre un appareil de pulvérisation comprenant un récipient contenant la composition transdermique, une buse de pulvérisation et un actionneur pour délivrer une dose mesurée de pulvérisation venant du récipient via la buse de pulvérisation.

14. Système de délivrance transdermique selon l'une quelconque des revendications 1 à 13 pour administration transdermique d'au moins un agent pharmacologique choisi parmi les hormones et les stéroïdes.

15. Système de délivrance transdermique selon la revendication 14, dans lequel l'administration transdermique s'effectue par application du médicament à une aire de surface dermique d'un sujet.

16. Système de délivrance transdermique selon la revendication 15, dans lequel le sujet a besoin du remplacement d'hormone mâle chez les hommes hypogonodiques déficients en testostérone, d'une thérapie de remplacement d'hormone femelle pour les femmes post-ménopausées, ou d'une thérapie de remplacement d'androgène pour les femmes dénuées de libido ou souffrant de dépression utilisant un androgène, une contraception masculine ou une contraception féminine.

17. Procédé pour la préparation d'un système de délivrance transdermique comprenant une composition pour administration à une aire de surface dermique d'un sujet, le procédé comprenant la combinaison d'au moins un agent pharmacologique choisi parmi les hormones et les stéroïdes, un renforçateur de pénétration comprenant du polyéthylène glycol ayant une masse moléculaire moyenne ne dépassant pas 300 et 70% à 95% en poids de la composition d'un solvant choisi par les alcanols en C₂ à C₄ et leurs mélanges.

18. Système de délivrance transdermique selon la revendication 1, dans lequel le PEG ayant une masse moléculaire moyenne ne dépassant pas 300 est le PEG 200 en une quantité allant de 0,1 à 40% en poids de la composition totale.

19. Système de délivrance transdermique selon la revendication 1, dans lequel le PEG ayant une masse moléculaire moyenne ne dépassant pas 300 est de formule H-[OCH₂CH₂]ₙ-OH où n est 4.
